# EUROPÄISCHE PATENTSCHRIFT

(11) **EP 2 536 446 B1**
(45) Veröffentlichungstag und Bekanntmachung des Hinweises auf die Patenterteilung: **26.03.2014**
(21) Anmeldenummer: 11717168.6
(22) Anmeldetag: 14.02.2011
(51) Int. Cl.: A61L 31/14

(54) **VORRICHTUNG ZUR ABDECKUNG UND/ODER REKONSTRUKTION EINER KNOCHENDEFEKTSTELLE UND VERFAHREN ZU DEREN HERSTELLUNG**
DEVICE FOR COVERING AND/OR RECONSTRUCTING A BONE DEFECT SITE, AND METHOD FOR PRODUCTION THEREOF
DISPOSITIF POUR LE RECOUVREMENT ET/OU LA RECONSTRUCTION D'UN DÉFAUT D'UN OS, ET SON PROCÉDÉ DE FABRICATION

(30) Priorität: 21.10.2010 DE 102010049809; 19.02.2010 DE 102010009333
(43) Veröffentlichungstag der Anmeldung: 26.12.2012
(73) Patentinhaber: Reoss GmbH, 70794 Filderstadt (DE)
(72) Erfinder: SEILER, Marcus, 70597 Stuttgart (DE)
(74) Vertreter: Schuster, Müller & Partner
(86) Internationale Anmeldenummer: PCT/DE2011/000131
(87) Internationale Veröffentlichungsnummer: WO 2011/100951

(56) Entgegenhaltungen:
- EP-A1- 0 809 979
- WO-A1-2010/019463
- DE-A1-102005 039 382
- US-A1- 2004 143 344

## Beschreibung

### Stand der Technik

Die Erfindung geht aus von einer Vorrichtung zur Abdeckung und/oder Rekonstruktion einer Knochendefektstelle, nach der Gattung des Anspruchs 1, und einem Verfahren zur Herstellung eines Aufsatzes einer Abdeckvorrichtung für eine Knochendefektstelle, nach der Gattung des Anspruchs 15.

Knochendefektstellen in Form von Ausnehmungen oder Höhlungen im körpereigenen Knochengewebe werden in der Knochenchirurgie, beispielsweise bei der Rekonstruktion von Knochen in der orthopädischen, neurochirurgischen oder plastischen Chirurgie oder bei kieferchirurgischen Operationen, oftmals mit Knochenaufbaumaterial gefüllt. In der Regel besteht das Knochenaufbaumaterial aus einer Mischung aus synthetischen Knochenersatzmaterial (z.B. Hydroxylapatitgranulat) und körpereigenen Knochenpartikeln. Damit das Knochenaufbaumaterial im Wesentlichen ausschließlich von der Knochenseite her knöchern durchwachsen wird, wird die Ausnehmung, wie in der Patentschrift DE 43 02 708 C2 beschrieben, mit einer Abdeckmembran verschlossen. Befestigt wird die Abdeckmembran mit Befestigungsnägeln am körpereigenen Knochen, wobei, da die Abdeckmembran aus einem flexiblen Material besteht, die Befestigung ein Höchstmaß an handwerklichem Geschick des Chirurgen erfordert.

Um diesen Nachteil einer fehlenden Stützfunktion der Abdeckmembran zu überwinden, wird in der Patentschrift US 48 16 339 eine Abdeckmembran beschrieben, die aus mehreren Schichten besteht, wobei diese Schichten nicht aus resorbierbaren Membranmaterial bestehen. Dabei ist es gegebenenfalls erforderlich, dass nach dem Ausheilen des Knochendefektes ein zweiter Eingriff notwendig ist, um körperfremdes Material zu entfernen.

In der Patentschrift DE 10 2005 039 382 B4 wird ein biodegradierbarer Hohlkörper, der insbesondere eine hohlzylindrische oder kegelzylindrische Form aufweist, vorgeschlagen. Der Hohlkörper weist in seinen Wandungen eine Mehrzahl von Öffnungen auf, durch die eine Aufnahme von Blut und damit der Aufbau von Eigenknochen möglich ist. Nachteilig ist hierbei, dass zum Einsetzen des Hohlkörpers eine zylindrische Bohrung mittels eines Bohrers in den vorhandenen Knochen eingebracht werden muss.

In der Offenlegungsschrift DE 10 2006 047 054 A1 wird ein Implantatlager vorgeschlagen, das sich durch eine hohe Passgenauigkeit und Stabilität auszeichnet, so dass der behandelnde Arzt es einfach handhaben und implantieren kann. Das aus Hydroxylapatit angefertigte Implantatlager, das zum Schutz der Schleimhaut vor mechanischen Einwirkungen und zum Schutz des Implantatlagers vor einwachsenden Gewebe von Seiten der Schleimhaut auf der der Schleimhaut zugewandten Seite eine dünne, insbesondere aus resorbierbaren Material bestehende, Membran aufweist, wird mit einem aufbauenden Fertigungsverfahren hergestellt, so dass die Materialbeschaffenheit eine "Gradientenstruktur" im Sinne einer insbesondere nach innen abnehmenden Dichte bildet. Dabei ist an der dem Knochen zugewandten Seite eine Bauweise mit einer insbesondere porösen Struktur und an der Außenseite des Implantatlagers, an der sich eine Struktur zur Halterung eines Zahnimplantats und/oder eines Zahnersatzes befindet, eine kompakte Bauweise vorgesehen.

### Die Erfindung und ihre Vorteile

Die erfindungsgemäße Vorrichtung zur Abdeckung und/oder Rekonstruktion einer Knochendefektstelle, wobei mit dem Begriff "Knochendefektstelle" eine Stelle eines (kranken) Knochens bezeichnet wird, die von der Form eines gesunden Knochens abweicht, mit dem kennzeichnenden Merkmal des Anspruchs 1, und das erfindungsgemäße Verfahren zur Herstellung eines Aufsatzes einer Abdeckvorrichtung für eine Knochendefektstelle, mit den kennzeichnenden Merkmalen des Anspruchs 15, haben demgegenüber den Vorteil, dass die Vorrichtung zur Abdeckung und/oder Rekonstruktion einer Knochendefektstelle aus einem Aufsatz (z.B. Formschale, starre Schale, Formkörper) und mindestens einem Fixiermittel zur Fixierung des Aufsatzes an einem Knochen besteht, wobei sich der Aufsatz durch eine formstabile (starre) Beschaffenheit auszeichnet und eine dem Knochendefekt zugewandte Wandung des Aufsatzes oder eine dem Knochendefekt abgewandte Wandung des Aufsatzes der Form des regenerierten Knochens, der durch seine Regeneration wieder die Form eines gesunden Knochens aufweist, entspricht.

Nach einer vorteilhaften Ausgestaltung der erfindungsgemäßen Vorrichtung bestehen der Aufsatz und/oder das Fixiermittel mindestens teilweise aus einem biokompatiblen Werkstoff. Der biokompatible Werkstoff kann biotolerant, bioinert und / oder bioaktiv sein.

Nach einer diesbezüglichen vorteilhaften Ausgestaltung der erfindungsgemäßen Vorrichtung ist der biokompatible Werkstoff mindestens teilweise ein autogener, syngener, allogener, xenogener, synthetischer oder alloplastischer Werkstoff.

Nach einer zusätzlichen vorteilhaften Ausgestaltung der erfindungsgemäßen Vorrichtung bestehen der Aufsatz und/oder das Fixiermittel mindestens teilweise aus einem biodegradierbaren Werkstoff.

Nach einer zusätzlichen vorteilhaften Ausgestaltung der erfindungsgemäßen Vorrichtung können der Aufsatz und/oder das Fixiermittel mindestens teilweise aus einem resorbierbaren Werkstoff bestehen. Vorteilhafterweise kann die Resorptionszeit der starren Schale durch deren Resorptionsgradienten gesteuert werden und/oder kann die Resorptionszeit auch weniger als sechs Monate betragen, so dass zeitnah das Implantat eingesetzt werden kann.

Nach einer zusätzlichen vorteilhaften Ausgestaltung der erfindungsgemäßen Vorrichtung bestehen der Aufsatz und/oder das Fixiermittel mindestens teilweise aus einem Polymer oder einer polymeren Verbindung.

Nach einer zusätzlichen vorteilhaften Ausgestaltung der erfindungsgemäßen Vorrichtung bestehen der Aufsatz und/oder das Fixiermittel mindestens teilweise aus Polylactid. Polylactide sind aus vielen chemisch aneinander gebundenen Milchsäuremoleküle aufgebaut und gehören zu den Polymeren. Der Vorteil von Polylactid-Kunststoffen, die auch Polymilchsäuren (PLA) genannt werden, ist, dass sie durch Wärmezufuhr verformbare Kunststoffe und biokompatibel sind.

Nach einer zusätzlichen vorteilhaften Ausgestaltung der erfindungsgemäßen Vorrichtung weist der Aufsatz eine konstante oder eine variierende Wandstärke auf.

Nach einer diesbezüglichen vorteilhaften Ausgestaltung der erfindungsgemäßen Vorrichtung sollte die Wandstärke mindestens 0,2 mm betragen, bevorzugt 0,5 mm, jedoch zumindest so viel, so dass sich eine Formstabilität der Formschale ergibt.

Nach einer zusätzlichen vorteilhaften Ausgestaltung der erfindungsgemäßen Vorrichtung ist das Fixiermittel ein Pin, eine Schraube, ein Nagel und/oder ein Knochenkleber.

Nach einer zusätzlichen vorteilhaften Ausgestaltung der erfindungsgemäßen Vorrichtung weist der Aufsatz mindestens eine Fräsung (Bohrung für das Fixiermittel) auf.

Nach einer diesbezüglichen vorteilhaften Ausgestaltung der erfindungsgemäßen Vorrichtung korrespondiert die Fräsung mit dem Fixiermittel.

Nach einer zusätzlichen vorteilhaften Ausgestaltung der erfindungsgemäßen Vorrichtung weist die dem Knochendefekt zugewandte Wandung eine Oberflächenkonditionierung auf.

Nach einer diesbezüglichen vorteilhaften Ausgestaltung der erfindungsgemäßen Vorrichtung kann die Oberfläche eine Mikrostrukturierung, Poren, Osteoblastenlockstoffe, Mittel zur Förderung des Knochenwachstums und/oder BMP-haltiges Knochenersatzmittel aufweisen.

Nach einer vorteilhaften Ausgestaltung des erfindungsgemäßen Verfahrens zur Herstellung eines Aufsatzes einer Abdeckvorrichtung für eine Knochendefektstelle, bei dem die rechnergestützte Formgebung (CAD) des Aufsatzes mit einer rechnergestützten Fertigung (CAM) zu CAD/CAM kombiniert wird, so dass ein am Computer entwickeltes Entwurfsmodell des Aufsatzes direkt elektronisch an die Fertigung übermittelt wird, bestehend aus folgenden Verfahrensschritten:
- Aufnahme eines Datensatzes, der die betroffene Knochendefektstelle in seiner Dreidimensionalität repräsentiert, mittels Tomografie oder ähnlichen bildgebenden Verfahren,
- Verwendung des Datensatzes zur Planung des Aufsatzes, der eine dem Knochendefekt abgewandte Wandung und eine dem Knochendefekt zugewandte Wandung aufweist, und mit mindestens einem Fixiermittel an einem Knochen fixierbar ist,
- Umsetzung der Planung des Aufsatzes in einen Planungsdatensatz und
- Zuführung des Planungsdatensatzes an ein computergesteuertes Fertigungsverfahren,
wobei der Aufsatz aus einem formstabilen Material gebildet wird und dessen dem Knochendefekt zugewandte Wandung oder dessen dem Knochendefekt abgewandte Wandung der Form des regenerierten Knochens entspricht, und die Aufnahme des Datensatzes, der die betroffene Knochendefektstelle in seiner Dreidimensionalität repräsentiert, mittels Computertomografie oder digitaler Volumentomografie erfolgt.

Nach einer zusätzlichen vorteilhaften Ausgestaltung des erfindungsgemäßen Verfahrens zur Herstellung eines Aufsatzes einer Abdeckvorrichtung für eine Knochendefektstelle wird im Fertigungsverfahren der Aufsatz mittels Fräsung gebildet.

Nach einer zusätzlichen vorteilhaften Ausgestaltung des erfindungsgemäßen Verfahrens zur Herstellung eines Aufsatzes einer Abdeckvorrichtung für eine Knochendefektstelle wird nach der Fertigung des Aufsatzes ein Reinigungs- und/oder Sterilisationsprozess durchgeführt.

Nach einer zusätzlichen vorteilhaften Ausgestaltung des erfindungsgemäßen Verfahrens zur Herstellung eines Aufsatzes einer Abdeckvorrichtung für eine Knochendefektstelle ist der Aufsatz in einer Vorrichtung zur Abdeckung und/oder Rekonstruktion einer Knochendefektstelle gemäß den Ansprüchen 1 bis 14 einsetzbar. Dadurch kann eine Vorrichtung zur Abdeckung und/oder Rekonstruktion einer Knochendefektstelle geschaffen werden, dessen Aufsatz und/oder Fixiermittel beispielsweise aus einem künstlichen Werkstoff und/oder aus einem Werkstoff autogener, synergener, allogener oder xenogener Herkunft menschliche und/oder tierische Knochen bzw. die menschliche, tierische oder künstliche Matrix eine Form auf, durch die der zwischen dem Knochen und der gewünschten Form des regenerierten Knochens befindlicher Bereich vollständig oder nahezu vollständig ausgefüllt wird. Hierzu wird dem Spender z.B. ein Knochenblock entnommen, der anschließend gegebenenfalls mittels CAD/CAM modelliert wird.

Weitere Vorteile und vorteilhafte Ausgestaltungen der Erfindung sind der nachfolgenden Beschreibung, der Zeichnung und den Ansprüchen entnehmbar.

### Zeichnung

Ausführungsbeispiele des Gegenstandes der Erfindung sind in der Zeichnung dargestellt und werden im Folgenden näher erläutert. Es zeigen:
- Fig. 1: eine Darstellung einer erfindungsgemäßen Vorrichtung zur Abdeckung und/oder Rekonstruktion einer Knochendefektstelle,
- Fig. 2: eine Darstellung einer anders geformten erfindungsgemäßen Vorrichtung zur Abdeckung und/oder Rekonstruktion einer Knochendefektstelle,
- Fig. 3: eine Darstellung einer anders geformten erfindungsgemäßen Vorrichtung zur Abdeckung und/oder Rekonstruktion einer Knochendefektstelle und
- Fig. 4: eine Darstellung einer anders geformten erfindungsgemäßen Vorrichtung zur Abdeckung und/oder Rekonstruktion einer Knochendefektstelle.

### Beschreibung des Ausführungsbeispiels

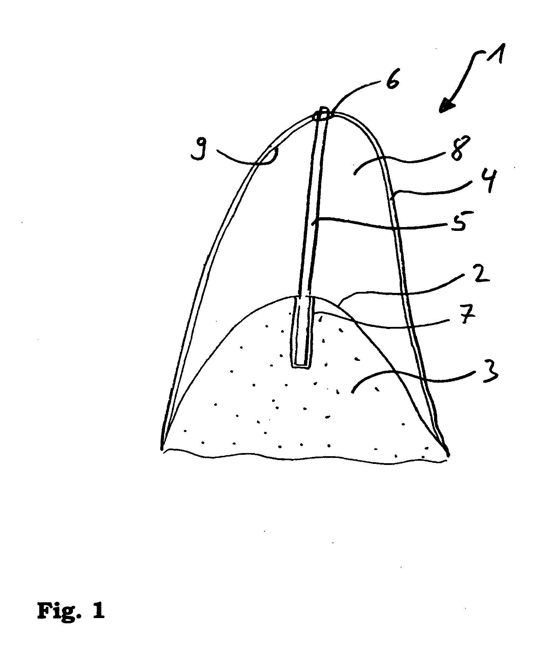

Fig. 1 zeigt eine Darstellung einer erfindungsgemäßen Vorrichtung 1 zur Abdeckung und/oder Rekonstruktion einer Knochendefektstelle 2 (Knochendefekt) eines Knochens, insbesondere eines Kieferknochens 3. Die Vorrichtung 1 besteht aus einem Aufsatz 4 und einem Fixiermittel 5, das in Fig. 1 als Pin dargestellt ist. Der Aufsatz 4 ist aus einem formstabilen Material, so dass er selbsttragend ist und keine zusätzliche Abstützung notwendig ist. Zur Fixierung des Aufsatz 4 (Formschale, starre Schale) wird das Fixiermittel 5 durch eine Bohrung 6 in den Aufsatz 4 geschoben und anschließend in die im Kieferknochen 3 angeordnete Bohrung 7 eingeführt. Die anschließende Fixierung des Aufsatzes 4 erfolgt über Ultraschall-Schweißen. Beim Ultraschall-Schweißen erzeugt bevorzugt ein Ultraschallgenerator eine genau definierte Frequenz, welche über eine Sonotrode gebündelt wird. Nach dem Aufsetzen des resorbierbaren Fixiermittels 5 (Pin) auf ein im Knochen vorgebohrtes Bohrloch (Bohrung 7) sorgt eine erzeugte Schwingung für eine Verflüssigung der Pinoberflächen an dessen Rändern, wodurch ein Eingleiten des Pins in das Bohrloch bewirkt wird. Durch die Änderung des Aggregatzustandes dringt der Pin auch in die knöchernen Hohlräume vor, die von einer gewöhnlichen Knochenschraube unerreichbar sind, so dass eine hohe initiale Festigkeit erzielt wird. Zudem verbindet sich der Pinkopf mit dem Aufsatz 4 und sorgt mit einem Verblockungsmechanismus für ein stabiles dreidimensionales Konstrukt. Beim Ultraschall-Schweißen wird somit das Fixiermittel 5 aufgeweicht, so dass es sich mit dem Kieferknochen 3 und dem Aufsatz 4 verbindet. Durch den fixierten Aufsatz 4 entsteht ein abgedichteter Innenraum 8 zwischen dem Kieferknochen 3 und dem Aufsatz 4, der durch die Regeneration des Knochens und/oder durch Einbringung von autogenen, syngenen, allogenen, xenogenen, synthetischen und/oder alloplastischen Material ausgefüllt wird, so dass der regenerierte Knochen oder das eingebrachte Material der Form der der Knochendefektstelle 2 zugewandten Wandung 9 des Aufsatzes 4 entspricht. Um den Regenerationsprozess des Kieferknochens 3 zu beschleunigen, kann die dem Knochendefekt zugewandte Wandung 9 des Aufsatzes 4 eine Oberflächenkonditionierung (z.B. eine Mikrostrukturierung, Poren, Osteoblastenlockstoffe, Mittel zur Förderung des Knochenwachstums und/oder BMP-haltiges Knochenersatzmittel) aufweisen.

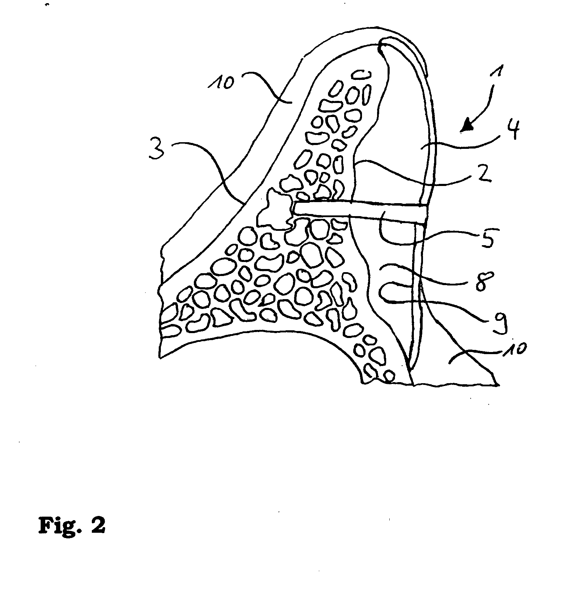

Fig. 2 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist zusätzlich das Zahnfleisch 10 angedeutet.

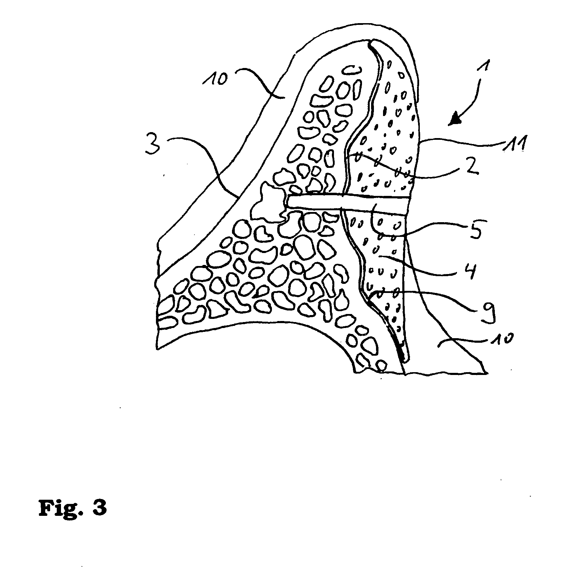

Fig. 3 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist der Aufsatz 4 als Formkörper z.B. aus menschlichen oder tierischen Knochen geformt und weist eine die dem Knochendefekt zugewandte Wandung 9, die an das Relief der Knochendefektstelle 2 angepasst ist, und eine dem Knochendefekt abgewandte Wandung 11, die der Form des regenerierten Knochens entspricht, auf.

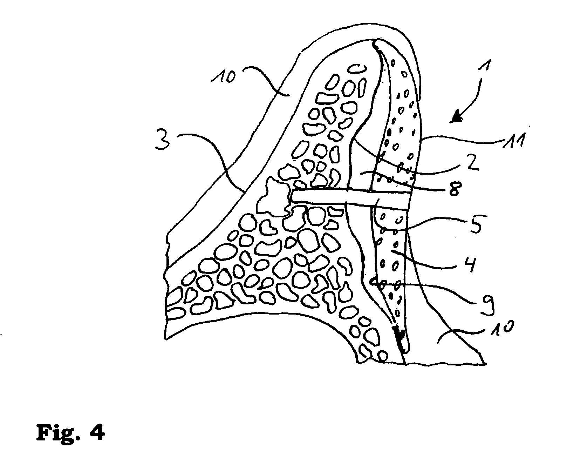

Fig. 4 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist der Aufsatz 4 als Formkörper z.B. aus menschlichen oder tierischen Knochen geformt und weist eine die dem Knochendefekt zugewandte Wandung 9 und eine dem Knochendefekt abgewandte Wandung 11, die der Form des regenerierten Knochens entspricht, auf. Zwischen der Wandung 9 und der Knochendefektstelle 2 befindet sich ein Innenraum 8, der durch die Regeneration des Knochens und/oder durch Einbringung von autogenen, syngenen, allogenen, xenogenen, synthetischen und/oder alloplastischen Material ausgefüllt wird.

Alle hier dargestellten Merkmale können sowohl einzeln als auch in beliebiger Kombination miteinander erfindungswesentlich sein.

### Bezugszahlenliste

- 1: Vorrichtung
- 2: Knochendefektstelle
- 3: Kieferknochen
- 4: Aufsatz
- 5: Fixiermittel
- 6: Bohrung
- 7: Bohrung
- 8: Innenraum
- 9: Wandung
- 10: Zahnfleisch
- 11: Wandung

## Patentansprüche

1. Vorrichtung (1) zur Abdeckung und/oder Rekonstruktion einer Knochendefektstelle (2),
- mit einem Aufsatz (4), der eine dem Knochendefekt abgewandte Wandung (11) und eine dem Knochendefekt zugewandte Wandung (9) aufweist,
- mit mindestens einem Fixiermittel (5) zur Fixierung des Aufsatzes (4) an einem Knochen,
**dadurch gekennzeichnet,**
**dass** der Aufsatz (4) aus einem formstabilen Material besteht und die dem Knochendefekt zugewandte Wandung (9) des Aufsatzes (4) oder die dem Knochendefekt abgewandte Wandung (11) des Aufsatzes (4) der Form des regenerierten Knochens entspricht.

2. Vorrichtung (1), nach Anspruch 1, **dadurch gekennzeichnet, dass** der Aufsatz (4) und/oder das Fixiermittel (5) mindestens teilweise aus einem biokompatiblen Werkstoff bestehen.

3. Vorrichtung (1), nach Anspruch 2, **dadurch gekennzeichnet, dass** der biokompatible Werkstoff mindestens teilweise ein autogener, syngener, allogener, xenogener, synthetischer oder alloplastischer Werkstoff ist.

4. Vorrichtung (1), nach einem der vorhergehenden Ansprüche, **dadurch gekennzeichnet, dass** der Aufsatz (4) und/oder das Fixiermittel (5) mindestens teilweise aus einem biodegradierbaren Werkstoff bestehen.

5. Vorrichtung (1), nach einem der vorhergehenden Ansprüche, **dadurch gekennzeichnet, dass** der Aufsatz (4) und/oder das Fixiermittel (5) mindestens teilweise aus einem resorbierbaren Werkstoff bestehen.

6. Vorrichtung (1), nach einem der vorhergehenden Ansprüche, **dadurch gekennzeichnet, dass** der Aufsatz (4) und/oder das Fixiermittel (5) mindestens teilweise aus einem Polymer oder einer polymeren Verbindung bestehen.

7. Vorrichtung (1), nach einem der vorhergehenden Ansprüche, **dadurch gekennzeichnet, dass** der Aufsatz (4) und/oder das Fixiermittel (5) mindestens teilweise aus Polylactid bestehen.

8. Vorrichtung (1), nach einem der vorhergehenden Ansprüche, **dadurch gekennzeichnet, dass** der Aufsatz (4) eine konstante oder eine variierende Wandstärke aufweist.

9. Vorrichtung (1), nach Anspruch 7, **dadurch gekennzeichnet, dass** die Wandstärke mindestens 0,2 mm beträgt.

10. Vorrichtung (1), nach einem der vorhergehenden Ansprüche, **dadurch gekennzeichnet, dass** das Fixiermittel (5) ein Pin, eine Schraube, ein Nagel und/oder ein Knochenkleber ist.

11. Vorrichtung (1), nach einem der vorhergehenden Ansprüche, **dadurch gekennzeichnet, dass** der Aufsatz (4) mindestens eine Fräsung aufweist.

12. Vorrichtung (1), nach Anspruch 10, **dadurch gekennzeichnet, dass** die Fräsung mit dem Fixiermittel (5) korrespondiert.

13. Vorrichtung (1), nach einem der vorhergehenden Ansprüche, **dadurch gekennzeichnet, dass** die dem Knochendefekt zugewandte Wandung (9) eine Oberflächenkonditionierung aufweist.

14. Vorrichtung (1), nach Anspruch 12, **dadurch gekennzeichnet, dass** die Oberflächenkonditionierung eine Mikrostrukturierung, Poren, Osteoblastenlockstoffe, Mittel zur Förderung des Knochenwachstums und/oder BMP-haltiges Knochenersatzmittel aufweist.

15. Verfahren zur Herstellung eines Aufsatzes (4) einer Abdeckvorrichtung für eine Knochendefektstelle (2), bestehend aus folgenden Verfahrensschritten:
- Aufnahme eines Datensatzes, der die betroffene Knochendefektstelle (2) in seiner Dreidimensionalität repräsentiert, mittels Tomografie oder ähnlichen bildgebenden Verfahren,
- Verwendung des Datensatzes zur Planung des Aufsatzes (4), der eine dem Knochendefekt abgewandte Wandung (11) und eine dem Knochendefekt zugewandte Wandung (9) aufweist, und mit mindestens einem Fixiermittel (5) an einem Knochen fixierbar ist,
- Umsetzung der Planung des Aufsatzes (4) in einen Planungsdatensatz und
- Zuführung des Planungsdatensatzes an ein computergesteuertes Fertigungsverfahren,
**dadurch gekennzeichnet,**
**dass** der Aufsatz (4) aus einem formstabilen Material gebildet wird und dessen dem Knochendefekt zugewandte Wandung (9) oder dessen dem Knochendefekt abgewandte Wandung (11) der Form des regenerierten Knochens entspricht.

16. Verfahren zur Herstellung eines Aufsatzes (4), nach Anspruch 15, **dadurch gekennzeichnet, dass** die Aufnahme des Datensatzes, der die betroffene Knochendefektstelle (2) in seiner Dreidimensionalität repräsentiert, mittels Computertomografie oder digitaler Volumentomografie erfolgt.

17. Verfahren zur Herstellung eines Aufsatzes (4), nach Anspruch 15 oder Anspruch 16, **dadurch gekennzeichnet, dass** im Fertigungsverfahren der Aufsatz (4) mittels Fräsung gebildet wird.

18. Verfahren zur Herstellung eines Aufsatzes (4), nach einem der Ansprüche 15 bis 17, **dadurch gekennzeichnet, dass** nach der Fertigung des Aufsatzes (4) ein Reinigungs- und/oder Sterilisationsprozess durchgeführt wird.

19. Verfahren zur Herstellung eines Aufsatzes (4), nach einem der Ansprüche 15 bis 18, **dadurch gekennzeichnet, dass** der Aufsatz (4) in einer Vorrichtung (1) zur Abdeckung und/oder Rekonstruktion einer Knochendefektstelle (2) gemäß den Ansprüchen 1 bis 14 einsetzbar ist.

## Claims

1. Device (1) for the covering and/or reconstruction of a bone defect site (2)
- with an attachment (4) which has a wall section (11) facing away from the bone defect and a wall section (9) facing the bone defect,
- with at least one fixing means (5) for fixing the attachment (4) to a bone,
**characterised in that**
the attachment (4) is made of a dimensionally stable material and the wall section (9) of the attachment (4) facing the bone defect, or the wall section (11) of the attachment (4) facing away from the bone defect corresponds to the shape of the regenerated bone.

2. Device (1) according to claim 1, **characterised in that** the attachment (4) and/or the fixing means (5) is/are at least partially made of a biocompatible material.

3. Device (1) according to claim 2, **characterised in that** the biocompatible material is at least partially an autogenic, syngeneic, allogeneic, xenogeneic, synthetic or alloplastic material.

4. Device (1) according to any one of the preceding claims, **characterised in that** the attachment (4) and/or the fixing means (5) is/are at least partially made of a biodegradable material.

5. Device (1) according to any one of the preceding claims, **characterised in that** the attachment (4) and/or the fixing means (5) is/are at least partially made of a resorbable material.

6. Device (1) according to any one of the preceding claims, **characterised in that** the attachment (4) and/or the fixing means (5) is/are at least partially made of a polymer or a polymer compound.

7. Device (1) according to any one of the preceding claims, **characterised in that** the attachment (4) and/or the fixing means (5) is/are at least partially made of polylactide.

8. Device (1) according to any one of the preceding claims, **characterised in that** the attachment (4) has a constant or varying wall thickness.

9. Device (1) according to claim 7, **characterised in that** the wall thickness is at least 0.2 mm.

10. Device (1) according to any one of the preceding claims, **characterised in that** the fixing means (5) is a pin, a screw, a nail and/or a bone adhesive.

11. Device (1) according to any one of the preceding claims, **characterised in that** the attachment (4) has at least one milling.

12. Device (1) according to claim 10, **characterised in that** the milling corresponds with the fixing means (5).

13. Device (1) according to any one of the preceding claims, **characterised in that** the wall section (9) facing the bone defect has surface conditioning.

14. Device (1) according to claim 12, **characterised in that** the surface conditioning comprises microstructuring, pores, osteoblast attractants, agents for promoting bone growth and/or bone substitute materials containing BMP.

15. Method of manufacturing an attachment (4) of a covering device for a bone defect site (2) comprising the following procedural steps:
- recording of a data set representing the three-dimensional state of the bone defect site (2) by means of tomography or a similar imaging procedure,
- use of the data set for planning the attachment (4), which has a wall section (11) facing away from the bone defect and a wall section (9) facing the bone defect and which can be fixed to a bone by way of a fixing means (5),
- implementing the planning of the attachment (4) in a planning data set and
- supplying the planning data set to a computer-controlled manufacturing process,
**characterised in that**
the attachment (4) is made of a dimensionally stable material and its wall section (9) facing the bone defect, or its wall section (11) facing away from the bone defect corresponds to the shape of the regenerated bone.

16. Method of manufacturing an attachment (4) according to claim 15, **characterised in that** the recording of the data set representing the three-dimensional state of the bone defect (2) involved takes place by means of computer tomography or digital volume tomography.

17. Method of manufacturing an attachment (4) according to claim 15 or claim 16, **characterised in that** in the manufacturing process the attachment (4) is produced by means of milling.

18. Method of manufacturing an attachment (4) according to any one of claims 15 to 17, **characterised in that** after manufacturing the attachment (4) a cleaning and/or sterilisation process is carried out.

19. Method of manufacturing an attachment (4) according to any one of claims 15 to 18, **characterised in that** the attachment (4) can be used in a device (1) for the covering and/or reconstruction of a bone defect site (2) according to claims 1 to 14.

## Revendications

1. Dispositif (1) destiné à recouvrir et/ou reconstruire un point endommagé d'un os (2),
- comprenant un chapeau (4) qui présente une paroi (11) détournée du point endommagé et une paroi (9) tournée vers le point endommagé,
- comprenant au moins un moyen de fixation (5) pour fixer le chapeau (4) sur un os,
**caractérisé en ce que**
le chapeau (4) est fabriqué dans un matériau à forme stable et la paroi (9) du chapeau (4) tournée vers le point endommagé ou la paroi (11) du chapeau (4) détournée du point endommagé correspond à la forme de l'os régénéré.

2. Dispositif (1) selon la revendication 1, **caractérisé en ce que** le chapeau (4) et/ou le moyen de fixation (5) sont fabriqués au moins partiellement dans un matériau biocompatible.

3. Dispositif (1) selon la revendication 2, **caractérisé en ce que** le matériau biocompatible est au moins partiellement un matériau autogène, syngène, allogène, xénogène, synthétique ou alloplastique.

4. Dispositif (1) selon l'une des revendications précédentes, **caractérisé en ce que** le chapeau (4) et/ou le moyen de fixation (5) sont fabriqués au moins partiellement dans un matériau biodégradable.

5. Dispositif (1) selon l'une des revendications précédentes, **caractérisé en ce que** le chapeau (4) et/ou le moyen de fixation (5) sont fabriqués au moins partiellement dans un matériau résorbable.

6. Dispositif (1) selon l'une des revendications précédentes, **caractérisé en ce que** le chapeau (4) et/ou le moyen de fixation (5) sont fabriqués au moins partiellement dans un polymère ou une liaison polymère.

7. Dispositif (1) selon l'une des revendications précédentes, **caractérisé en ce que** le chapeau (4) et/ou le moyen de fixation (5) sont fabriqués au moins partiellement en polylactide.

8. Dispositif (1) selon l'une des revendications précédentes, **caractérisé en ce que** le chapeau (4) présente une épaisseur de paroi constante ou variable.

9. Dispositif (1) selon la revendication 7, **caractérisé en ce que** l'épaisseur de paroi fait au moins 0,2 mm.

10. Dispositif (1) selon l'une des revendications précédentes, **caractérisé en ce que** le moyen de fixation (5) est une broche, une vis, un clou et/ou une colle pour os.

11. Dispositif (1) selon l'une des revendications précédentes, **caractérisé en ce que** le chapeau (4) présente au moins un fraisage.

12. Dispositif (1) selon la revendication 10, **caractérisé en ce que** le fraisage correspond au moyen de fixation (5).

13. Dispositif (1) selon l'une des revendications précédentes, **caractérisé en ce que** la paroi (9) tournée vers le point endommagé présente un conditionnement de surface.

14. Dispositif (1) selon la revendication 12, **caractérisé en ce que** le conditionnement de surface est une microstructuration, des pores, des substances attractives d'ostéoblastes, des moyens pour favoriser la croissance osseuse et/ou des produits de substitution osseuse contenant une BMP.

15. Procédé de fabrication d'un chapeau (4) d'un dispositif destiné à recouvrir un point endommagé d'un os (2), composé des étapes suivantes :
- enregistrement de données qui représentent le point endommagé d'un os (2) dans sa tridimensionnalité, par tomographie ou procédé similaire fournissant des images,
- emploi des données pour planifier le chapeau (4) qui présente une paroi (11) détournée du point endommagé et une paroi (9) tournée vers le point endommagé, et qui peut être fixé sur un os par au moins un moyen de fixation (5),
- la mise en place de la planification du chapeau (4) dans des données de planification et
- l'insertion des données de planification dans un procédé de fabrication piloté par ordinateur,
**caractérisé en ce que**
le chapeau (4) est en matériau à forme stable et sa paroi (9) tournée vers le point endommagé ou sa paroi (11) détournée du point endommagé correspond à la forme de l'os régénéré.

16. Procédé de fabrication d'un chapeau (4) selon la revendication 15, **caractérisé en ce que** l'enregistrement des données qui représentent le point endommagé d'un os (2) dans sa tridimensionnalité, est effectué par tomographie par ordinateur ou tomographie volumique numérique.

17. Procédé de fabrication d'un chapeau (4) selon la revendication 15 ou 16, **caractérisé en ce que** le chapeau (4) est formé par un fraisage au cours du procédé de fabrication.

18. Procédé de fabrication d'un chapeau (4) selon l'une des revendications 15 à 17, **caractérisé en ce qu'**un procédé de nettoyage et/ou de stérilisation est effectué après la fabrication du chapeau (4).

19. Procédé de fabrication d'un chapeau (4) selon l'une des revendications 15 à 18, **caractérisé en ce que** le chapeau (4) peut être intégré dans un dispositif (1) destiné à recouvrir et/ou reconstruire un point endommagé d'un os (2) selon l'une des revendications 1 à 14.
